(19) Europäisches Patentamt
European Patent Office
Office européen des brevets

(11) **EP 3 979 834 B1**

(12) **FASCICULE DE BREVET EUROPEEN**

(45) Date de publication et mention
de la délivrance du brevet:
**23.10.2024 Bulletin 2024/43**

(21) Numéro de dépôt: **20733692.6**

(22) Date de dépôt: **29.05.2020**

(51) Classification Internationale des Brevets (IPC):
*A23L 33/14* (2016.01)    *A61K 36/064* (2006.01)
*A61P 31/04* (2006.01)    *A61P 13/00* (2006.01)

(52) Classification Coopérative des Brevets (CPC):
**A23L 33/14; A61K 36/064; A61P 31/04;**
Y02A 50/30

(86) Numéro de dépôt international:
**PCT/EP2020/065046**

(87) Numéro de publication internationale:
**WO 2020/245057 (10.12.2020 Gazette 2020/50)**

(54) **COMPOSITION COMPRENANT UNE LEVURE POUR LA PREVENTION DE LA CYSTITE SIMPLE ET/OU RECIDIVANTE**

ZUSAMMENSETZUNG ENTHALTEND EINE HEFE ZUR BEHANDLUNG VON WIEDERKEHRENDER CYSTITIS

COMPOSITION COMPRISING A YEAST FOR TREATING SIMPLE OR RECURRENT CYSTITIS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **04.06.2019 FR 1905888**

(43) Date de publication de la demande:
**13.04.2022 Bulletin 2022/15**

(73) Titulaire: **Lesaffre et Compagnie**
**75001 Paris (FR)**

(72) Inventeurs:
• **DECHERF, Amélie**
**59910 Bondues (FR)**
• **BALLET, Nathalie**
**59700 Marcq-en-Baroeul (FR)**

(74) Mandataire: **Plasseraud IP**
**104 Rue de Richelieu**
**CS92104**
**75080 Paris Cedex 02 (FR)**

(56) Documents cités:
**WO-A1-2014/009656     WO-A2-2009/103884**

• **MADDEN-FUENTES RAMIRO J ET AL: "Efficacy of Fluoroquinolone/Probiotic Combination Therapy for Recurrent Urinary Tract Infection in Children: A Retrospective Analysis", CLINICAL THERAPEUTICS, EXCERPTA MEDICA, PRINCETON, NJ, US, vol. 37, no. 9, 29 July 2015 (2015-07-29), pages 2143 - 2147, XP029267367, ISSN: 0149-2918, DOI: 10.1016/J.CLINTHERA.2015.06.018**
• **ANUM KHAN ET AL: "Update on Associated Risk Factors, Diagnosis, and Management of Recurrent Urinary Tract Infections in Children", JOURNAL OF THE PEDIATRIC INFECTIOUS DISEASES SOCIETY, vol. 8, no. 2, 23 July 2018 (2018-07-23), pages 152 - 159, XP055680219, DOI: 10.1093/jpids/piy065**
• **CAROL A. KAUFFMAN ET AL: "Candida Urinary Tract Infections-Diagnosis", CLINICAL INFECTIOUS DISEASES, vol. 52, no. suppl_6, 15 May 2011 (2011-05-15), US, pages S452 - S456, XP055680368, ISSN: 1058-4838, DOI: 10.1093/cid/cir111**

**Description**

DOMAINE TECHNIQUE DE L'INVENTION

La présente invention concerne le domaine des infections urinaires. Elle concerne plus particulièrement une levure pour prévenir la cystite simple et/ou la cystite récidivante.

ARRIERE PLAN TECHNOLOGIQUE

Les infections urinaires sont la première cause d'infection bactérienne dans le monde. Le sexe et l'âge sont des facteurs de risque importants pour contracter une infection urinaire. De façon générale et toutes catégories d'âges confondues, les femmes sont plus à risque de développer une infection urinaire. Ainsi, plus de 50% des femmes auront une infection urinaire au moins une fois au cours de leur vie et jusqu'à 30% de ces femmes souffriront d'infections urinaires récidivantes. Quant aux hommes, 10 à 15% d'entre eux souffriront d'au moins un épisode infectieux.

En milieu hospitalier, les infections urinaires représentent la deuxième infection nosocomiale après les infections pulmonaires. La cause la plus commune étant la sonde urinaire.

Les infections urinaires peuvent aussi être classées par rapport aux complications. On parle d'infections urinaires non compliquées lorsqu'il n'y a pas d'anomalies de structure ou de fonction du tractus urinaire, qu'il n'y pas d'infection au niveau rénal (pas de néphropathie) et en l'absence de comorbidité (autres maladies comme le diabète, l'hypertension, etc). On estime qu'il s'agit d'une infection urinaire compliquée lorsque les reins sont touchés, avec des caractéristiques cliniques associés à d'autres maladies comme les tumeurs, des causes métaboliques, de l'insuffisance rénale. De même, l'infection urinaire chez l'homme est classée comme à risque de complication car pratiquement toujours liée à une cause anatomique.

Les infections urinaires dans la population sont généralement causées par un seul micro-organisme : le pathogène *Escherichia coli* dans plus de 80% des infections urinaires. Dans 10 à 15% des infections, le pathogène *Staphylococcus saprophyticus* en est responsable.

L'antibiorésistance en particulier chez les souches d'*Escherichia coli* uropathogènes (UPEC) est croissante et préoccupante. Cela incite les scientifiques et les professionnels de santé à identifier de nouvelles stratégies thérapeutiques. C'est ainsi que l'utilisation de solutions naturelles, sans effets indésirables, est présentée comme une solution alternative aux antibiotiques.

Le document Ramiro J. Madden-Fuentes et al. (Clinical Therapeutics/Volume 37, Number 9, 2015) décrit l'administration à des enfants ayant des infections urinaires récurrentes (rUTI) d'une combinaison d'une fluoroquinolone avec *Saccharomyces cerevisiae var. boulardii.* Cette combinaison permet de diminuer la récurrence des épisodes d'infection.

Le document Alkil & al. (Pediatr. Nephrol, 2006) a évalué l'influence de la prise orale de *Saccharomyces cerevisiae var. boulardii* sur le nombre total de colonies *E. coli* dans le colon d'enfants. Il a été démontré que l'administration de *S. boulardii* permettait de diminuer significativement les colonies d'*E. coli* dans le colon tout en augmentant significativement le nombre de colonies de *S. boulardii.* Les auteurs concluent que *S. boulardii* peut être efficace pour réduire le nombre total de colonies *E. coli* dans les selles. Il n'est toutefois pas précisé si cette levure a un effet sur la prévention des infections urinaires.

Il existe donc un réel besoin de disposer de nouvelles solutions naturelles pour prévenir les infections urinaires.

RESUME DE L'INVENTION

La demanderesse a ainsi mis en évidence de manière surprenante que l'utilisation de levures permettait de prévenir l'apparition de cystites.

Ainsi, selon un premier aspect de l'invention, la présente invention concerne une composition comprenant au moins une levure pour son utilisation dans la prévention de la cystite simple et/ou de la cystite récidivante, caractérisée en ce que la levure étant sous la forme d'une levure vivante, d'une levure morte, d'écorces de levure, de fractions purifiées d'écorces de levure ou leurs mélanges, et en ce que la levure est la levure obtenue par culture de la souche *Saccharomyces cerevisiae* déposée à la CNCM le 17 octobre 2007 sous le numéro I-3856.

Selon un deuxième aspect, la présente invention concerne une composition comprenant au moins une levure pour son utilisation dans la prévention de la cystite simple et/ou de la cystite récidivante, caractérisée en ce que la levure étant sous la forme d'une levure vivante, d'une levure morte, d'écorces de levure, de fractions purifiées d'écorces de levure ou leurs mélanges, et en ce que la levure est la levure obtenue par culture de la souche *Saccharomyces cerevisiae* var. *boulardii* déposée à la CNCM le 21 août 2007 sous le numéro I-3799.

Selon un troisième aspect, l'invention concerne un dispositif médical comprenant une composition telle que définie ci-dessus pour son utilisation dans la prévention de la cystite simple et/ou de la cystite récidivante.

DESCRIPTION DETAILLEE DE L'INVENTION

La présente invention se rapporte à une composition comprenant au moins une levure telle que définie ci-dessus pour son utilisation dans la prévention de la cystite simple et/ou de la cystite récidivante.

Par « cystite simple », on entend une infection urinaire non à risque de complication. Cela concerne uniquement la femme sans facteur de risque en présence des seuls symptômes caractéristiques de la cystite tels qu'un besoin urgent ou plus fréquent d'uriner, une sensation de brûlure, ou des picotements, en l'absence de fièvre.

Par « cystite récidivante », on entend une succession d'épisodes infectieux conduisant à une cystite simple. Pour pouvoir parler de « cystite récidivante », le nombre d'épisodes infectieux doit être supérieur ou égal à quatre par an.

Le terme « levure » désigne une souche de levure, une levure obtenue par culture de la souche de levure, la levure pouvant être sous forme vivante ou morte, et sous forme de dérivés de levure tels que les écorces de levure et les fractions purifiées d'écorces de levure, ou leurs mélanges.

L'expression « souche de levure » désigne une population relativement homogène de cellules de levure obtenues par culture (ou multiplication) de la souche de départ.

Une souche de levure est obtenue à partir d'un clone, un clone étant une population de cellules de levure obtenue à partir d'une seule cellule de levure.

La culture d'une souche de levure peut s'effectuer par n'importe quelle méthode appropriée. Les procédés de culture de levure sont connus dans l'art antérieur, et l'homme du métier sait optimiser les conditions de culture pour chaque souche en fonction de sa nature. Une levure est obtenue par multiplication d'une souche de levure dans un milieu de culture, par exemple comme décrit dans le livre de référence « Yeast Technology », 2ème édition, 1991, G. Reed et T.W. Nagodawithana, publié par Van Nostrand Reinhold, ISBN 0-442-31892-8.

Ainsi, par exemple, à l'échelle industrielle, des cellules de levure utilisables dans le contexte de la présente invention, peuvent être obtenues par un procédé comprenant les étapes suivantes :

- culture d'une souche de levure dans un milieu de culture en plusieurs stades, d'abord en semi-anaérobiose, puis en aérobiose (milieu/atmosphère riche en oxygène) pour obtenir une multiplication des cellules de levure de départ ; et
- séparation, par centrifugation, des cellules de levure ainsi produites pour obtenir une crème de levure liquide contenant entre 12% et 25% de matière sèche levure.

La levure ainsi obtenue est une levure vivante. Une levure vivante ou levure active désigne une population de cellules de levure dont le métabolisme est actif.

Dans le contexte de la présente invention, la levure vivante se présente sous forme d'une levure sèche. Une levure sèche est caractérisée par une teneur faible en eau, et comprend généralement un taux de matière sèche levure supérieur à 90%, de préférence un taux de matière sèche compris entre 93% et 96%. Un des avantages d'une levure sèche est sa longue durée de conservation.

Ainsi le procédé de production de cellules de levures peut en outre comprendre une étape ultérieure de séchage des cellules, pour obtenir une levure sous forme sèche. Le séchage peut par exemple être un séchage par lyophilisation, un séchage par lit fluidisé, un séchage en tambour ou un séchage par atomisation.

Selon un autre mode de réalisation, la souche de levure est utilisée sous forme de levure morte. Une levure morte peut également être appelée « levure désactivée » ou « levure inactive » ou « levure inactivée ». Il s'agit d'une levure dont le métabolisme est irrémédiablement arrêté.

Une levure morte peut être obtenue par des techniques bien connues de l'homme du métier, telles qu'un traitement thermique de la levure, un traitement consistant à soumettre la levure à plusieurs cycles de congélation et décongélation successives, un traitement par irradiation, un traitement par atomisation ou une combinaison de ces traitements. Une levure inactive se trouve généralement sous forme sèche.

Dans un autre mode de réalisation, c'est un dérivé de cellules de levure qui est utilisé dans le contexte de la présente invention. Ce dérivé est choisi parmi les écorces de levure et les fractions purifiées d'écorces de levure.

Les écorces de levure correspondent à la fraction insoluble des cellules de levure, c'est-à-dire la paroi et la membrane plasmique des cellules de levures.

De façon classique, les écorces de levure sont obtenues par un procédé comprenant une étape d'autolyse ou d'hydrolyse enzymatique des cellules de levure suivie d'une étape de séparation de la fraction soluble de la fraction insoluble, la fraction insoluble isolée correspondant aux écorces de levure et la fraction soluble correspondant aux extraits de levure. La fraction insoluble peut ensuite être séchée. Les écorces de levure peuvent se présenter sous forme liquide, sous forme sèche ou sous forme visqueuse. On considère qu'elles sont sous forme sèche lorsque leur teneur en matière sèche est d'au moins 85%. Au contraire, si leur teneur en matière sèche est inférieure à 20% en masse, on considère qu'elles sont sous forme liquide. A partir de 20% et en-dessous de 85% en masse de matière sèche, on considère que les écorces de levure sont sous forme visqueuse. Les écorces de levure sont de préférence utilisées sous forme sèche. Le procédé d'obtention des écorces de levure est tel qu'il conserve les polysaccharides de structure de la paroi cellulaire, c'est-à-dire les β-glucanes et les mannanes, ces mannanes étant sous forme de mannoprotéines. Les procédés d'obtention d'écorces de levure sont connus dans l'art antérieur (voir par exemple l'ouvrage de référence "Yeast Technology", 2ème édition, 1991, G. Reed et T.W. Nogodawithana, publié par Van Nostrand Reinhold, New York, ISBN 0-442-31892-8).

Les fractions purifiées des écorces de levure concernent donc particulièrement les glucanes et les mannanes.

Les mannoprotéines et les glucanes sont obtenus par des procédés classiques bien connus de l'homme de l'art. Leur extraction des écorces peut être effectuée par solubilisation sélective, via une hydrolyse par voie chimique, enzymatique ou physico-chimique.

En particulier, le terme "β-glucanes pariétaux" désigne les β-glucanes de la paroi des cellules de levure, qui sont essentiellement des polymères de glucose dont les unités glucose de la chaîne principale sont reliées par des liaisons β-1,3 et dont les ramifications sont reliées par des liaisons β-1,6. Les β-glucanes de levure sont insolubles et présentent une viscosité faible. L'homme du métier sait comment extraire les β-glucanes de la paroi des cellules de levure. Une méthode commune comprend des extractions successives à chaud avec une base et avec un acide (comme l'acide acétique), suivies par des lavages aqueux pour éliminer tout composé soluble des écorces de levure, et la récupération du matériel insoluble constitué des β-glucanes pariétaux.

Le terme "mannoprotéines pariétales" désigne des polysaccharides de levure et plus précisément des copolymères d'oses neutres ou acides (à 5 ou 6 atomes de carbone), liés entre eux par des liaisons glycosidiques et liés à des protéines. Dans les parois de levure, les glucanes constituent un réseau sur lequel sont liés de manière covalente d'autres glucanes, la chitine, et les mannoprotéines. Les mannoprotéines sont fixées aux glucanes par l'intermédiaire d'un glycosylphosphate contenant une chaîne de 5 résidus mannose. L'homme du métier sait comment extraire les mannoprotéines de la paroi des cellules de levure. Une méthode commune comprend une digestion enzymatique des parois de levure avec une préparation de β-glucanases, suivie par la séparation de l'hydrolysat par centrifugation, et une purification par ultrafiltration. Une autre méthode est basée sur une extraction chimique effectuée à chaud.

La présente invention concerne ainsi une composition comprenant au moins une levure selon l'invention pour son utilisation dans la prévention de la cystite simple et/ou de la cystite récidivante. Comme déjà indiqué, la levure selon l'invention est choisie parmi une levure vivante, une levure morte, des écorces de levure, des fractions purifiées d'écorces de levure ou leurs mélanges. Sans vouloir être liés par aucune théorie, les présents inventeurs sont d'avis que l'administration d'une levure selon l'invention permettrait de moduler les facteurs de virulence du pathogène conduisant à une réduction de sa pathogénicité. Également, les inventeurs pensent qu'une co-agrégation levure - pathogène entrerait en jeu, favorisant ainsi l'élimination du pathogène.

Selon un mode de réalisation préféré de l'invention, la cystite simple et /ou récidivante est une cystite induite par des bactéries *Escherichia coli* uropathogènes dites UPEC pour « Uropathogenic *Escherichia coli* ».

De manière surprenante, les inventeurs ont découvert que l'administration d'une composition comprenant la levure selon l'invention avait pour effet de réduire le portage des UPEC dans les voies urinaires comme le démontreront les exemples.

Le terme « portage » désigne la présence et/ou la multiplication d'un pathogène au niveau d'un site anatomique qu'il soit colonisant ou infectant.

Selon un autre mode de réalisation de l'invention, la levure selon l'invention est obtenue par culture de la souche *Saccharomyces cerevisiae* déposée le 17 octobre 2007 en vertu du traité de Budapest à la Collection Nationale de Cultures de Microorganismes (CNCM), Institut Pasteur, 25 rue du Docteur Roux, 75724 Paris cedex 15, sous le numéro I-3856.

Cette souche de levure *Saccharomyces cerevisiae* a précédemment été décrite, par le présent Déposant lui-même, dans le document WO 2009/103884, où elle est décrite comme étant utile dans la prévention et/ou le traitement de pathologies, désordres ou troubles de l'intestin ; ainsi que dans le document WO 2014/009656 où elle est décrite comme étant utile dans la prévention et/ou le traitement des mycoses vaginales et en particulier les candidoses vaginales ou vulvovaginales.

Selon un autre mode de réalisation de l'invention, la levure est obtenue par culture de la souche *Saccharomyces cerevisiae* déposée le 21 août 2007 en vertu du traité de Budapest à la Collection Nationale de Cultures de Microorganismes (CNCM), Institut Pasteur, 25 rue du Docteur Roux, 75724 Paris cedex 15, sous le numéro I-3799.

Cette souche *Saccharomyces cerevisiae var boulardii* a précédemment été décrite, par le présent Déposant lui-même, dans le document WO 2009/103884, où elle est présentée comme étant utile dans la prévention et/ou le traitement de pathologies, désordres ou troubles de l'intestin.

Le microbiote correspond à l'ensemble des micro-organismes peuplant un microbiome, un environnement spécifique. Il n'existe pas un mais plusieurs microbiotes dont la composition varie selon la zone du corps. Sa composition varie par exemple en fonction du milieu aérobie ou anaérobie, en fonction de l'acidité du microbiome, en fonction de la sécheresse ou de l'humidité du milieu ou encore s'il s'agit d'une zone grasse ou non. On différencie ainsi le microbiote cutané, le microbiote vaginal, le microbiote urinaire, le microbiote respiratoire, le microbiote ORL et le microbiote intestinal qui est le plus important. Il est donc important de rappeler que les pathogènes incriminés dans les pathologies du tractus urinaire et ceux de la muqueuse vaginale ou de la muqueuse intestinale sont différents. Ainsi, le comportement des pathogènes et des probiotiques vis-à-vis de la muqueuse vaginale ou de la muqueuse intestinale n'est pas transposable à celui adopté vis-à-vis du tractus urinaire, l'environnement de ces muqueuses étant différent.

Selon un mode de réalisation particulier de l'invention, la levure selon l'invention est sous forme de levure sèche.

Selon un autre mode de réalisation de l'invention, la composition comprenant au moins une levure pour son utilisation dans la prévention de la cystite simple et/ou récidivante est destinée à une administration par voie orale ou voie locale.

La présente invention a particulièrement pour objet la composition telle que définie ci-dessus, pour son utilisation dans la prévention de la cystite simple et/ou récidivante, caractérisée en ce que la composition est sous forme de gélule, pastille, pilule, comprimé, capsule, poudre, suspension, solution liquide, granule, ovule, gel ou crème. L'homme de l'art sait sélectionner les véhicules et excipients les plus appropriés à la préparation d'un type donné de formulation.

Une composition selon l'invention peut en outre comprendre des additifs comme des conservateurs, des édulcorants, des arômes, des agents viscosants, des colorants, des agents humectants, des agents de désintégration, des accélérateurs d'absorption, des agents lubrifiants, etc.

La présente invention a par ailleurs pour objet une composition telle que définie ci-dessus, pour son utilisation dans la prévention de la cystite simple et/ou de la cystite récidivante, caractérisée en ce que la composition est une composition alimentaire ou un complément alimentaire.

La composition alimentaire désigne n'importe quel aliment, boisson, ou confiserie.

La composition alimentaire peut par exemple être une boisson, une barre de céréales, un chewing-gum, un produit laitier.

On entend par « complément alimentaire » une denrée alimentaire dont le but est de compléter le régime alimentaire normal et qui constitue une source concentrée de nutriments ou d'autres substances ayant un effet nutritionnel ou physiologique, seuls ou combinés.

Un complément alimentaire est commercialisé sous forme de dose, par exemple les formes de présentation telles que gélule, pastille, comprimé, pilule et autres formes similaires, sachet de poudre, ampoule de liquide, flacon muni d'un compte-goutte et autres formes analogues de préparations liquides ou en poudres destinées à être prises en unités mesurées de faible quantité.

La présente invention a également pour objet une composition telle que définie ci-dessus pour son utilisation dans la prévention de la cystite simple et/ou de la cystite récidivante, caractérisée en ce qu'elle comprend en outre un autre ingrédient actif.

L'autre ingrédient actif est à base de plantes choisi parmi un extrait de canneberge, de myrtilles, airelle rouge, arbousier, bruyère ou leurs mélanges lorsque la composition est destinée à un usage oral.

Dans un mode de réalisation particulier de l'invention, l'autre ingrédient actif n'est pas une levure.

La présente invention a par ailleurs pour objet un dispositif médical caractérisé en ce qu'il comprend une composition telle que décrite ci-dessus, pour son utilisation dans la prévention de la cystite simple et/ou récidivante.

Selon un mode avantageux de réalisation de l'invention, le dispositif médical est un tampon ou une serviette périodique, une crème, un gel, un ovule, un comprimé, une gélule, une pastille, une pilule et autres formes similaires.

La présente invention va maintenant être illustrée à l'aide des exemples et des figures qui suivent, qui sont donnés à titre d'illustration, et ne sont nullement limitatifs.

BREVE DESCRIPTION DES FIGURES

[Fig. 1] représente la quantité en UFC de la souche UPEC dans les sections intestinales pour chacun des groupes testés.

[Fig. 2] représente la charge bactérienne en UFC/mL (Log10) dans l'urine pour chaque groupe testé.

[Fig. 3] représente la charge bactérienne en UFC/mL (Log10) dans la vessie pour chaque groupe testé.

[Fig. 4] représente la charge bactérienne en UFC/mL (Log10) dans les reins pour chaque groupe testé.

EXEMPLES

**Exemple 1 : Essais de co-agrégation de levures avec des souches UPEC**

*Matériel et méthodes*

Souches UPEC

Deux souches *E. coli* uropathogènes sont utilisées dans le présent exemple et ont été isolées à partir d'urine obtenue à mi-miction.

La première souche *E.coli* utilisée, dite UPEC1, est sensible à tous les antibiotiques.

La deuxième souche *E. coli* utilisée, dite UPEC2, est multirésistante aux antibiotiques.

Conditions de croissance des souches UPEC

Les souches *Escherichia coli* sont cultivées une nuit à 37°C sur gélose Mueller-Hinton avant les essais. Les souches sont ensuite récoltées par centrifugation pendant 5 minutes à 11000 rpm, lavées 2 fois avec une solution stérile de tampon phosphate salin, dit PBS, la concentration étant ajustée à la concentration désirée et re-suspendue dans le même tampon.

Levures testées

Les levures testées pour chacune des deux souches UPEC sont les suivantes :

- Levure inactive *Saccharomyces cerevisiae* CNCM I-3856 (IY) ayant un taux de matière sèche $\geq$ 94% et un pH compris entre 5,5 et 6,3,
- Levure vivante *Saccharomyces cerevisiae* CNCM I-3856 (GI) à une teneur $\geq$ 5.10$^9$ UFC/g,
- Levure vivante *Saccharomyces cerevisiae var. boulardii* CNCM I-3799 (SCB) à une teneur $\geq$ 2.10$^{10}$ UFC/g,
- Glucanes pariétaux (WG) ayant un taux de beta-glucanes $\geq$ 50%.

Avant les essais, les levures sont réhydratées avec le tampon PBS.

Protocole de co-agrégation

Les souches UPEC ($1 \times 10^9$ cellules/mL) en solution tampon de PBS sont ajoutées à un volume égal à celui de la levure (10mg/mL).

Ce mélange est mélangé par vortex pendant au moins 10 secondes et déposé dans une plaque à 24 puits pendant 4 heures à 37°C sous agitation, à raison de 1mL par puit.

La suspension est ensuite observée au microscope à lumière transmise inversée pour évaluer le degré d'agrégation et donner un score selon l'échelle suivante :

0 = pas d'agrégation

1 = petits agrégats comprenant de petites grappes visibles

2 = agrégats comprenant un nombre plus important de microorganismes, installées au centre du puit

3 = amas macroscopiquement visible comprenant des groupes plus larges installés au centre du puit

4 = score maximum alloué pour décrire un important amas macroscopiquement visible au centre du puit.

De manière parallèle, est également testée la capacité d'auto-agrégation des différentes levures entre elles selon le même protocole ci-dessus mais sans ajout des souches UPEC dans le mélange. Les levures sont testées individuellement.

*Résultats*

Les résultats sont issus de 3 essais indépendants les uns des autres, chacun des essais étant réalisé avec des échantillons dupliqués (ci-après dp).

Les résultats des tests d'auto et co-agrégation sont donnés dans les tableaux 1 et 2 ci-dessous.

[Tableau 1]

| Essais | dp | UPEC1 | IY | GI | SCB | WG | UPEC1 + IY | UPEC1 + GY | UPEC1 + SCB | UPEC1 + WG |
|---|---|---|---|---|---|---|---|---|---|---|
| 1 | 1 | 0 | 4 | 0 | 1 | 4 | 3 | 3 | 4 | 4 |
|  | 2 | 0 | 4 | 0 | 1 | 4 | 3 | 3 | 4 | 4 |
| 2 | 1 | 0 | 4 | 0 | 1 | 4 | 3 | 3 | 4 | 4 |
|  | 2 | 0 | 4 | 0 | 1 | 4 | 3 | 3 | 4 | 4 |
| 3 | 1 | 0 | 4 | 0 | 1 | 4 | 3 | 4 | 4 | 4 |
|  | 2 | 0 | 4 | 0 | 1 | 4 | 3 | 4 | 4 | 4 |
| Score moyen |  | 0 | 4 | 0 | 1 | 4 | 3 | 3,33 | 4 | 4 |

**Résultats des essais d'auto et co-agrégation entre UPEC1 et les levures**

[Tableau 2]

| Essais | dp | UPEC2 | IY | GI | SCB | WG | UPEC2 + IY | UPEC2 + GY | UPEC2 + SCB | UPEC2 + WG |
|---|---|---|---|---|---|---|---|---|---|---|
| 1 | 1 | 0 | 4 | 0 | 1 | 4 | 3 | 4 | 4 | 4 |
|  | 2 | 0 | 4 | 0 | 1 | 4 | 3 | 4 | 4 | 4 |
| 2 | 1 | 0 | 4 | 0 | 1 | 4 | 3 | 4 | 4 | 4 |
|  | 2 | 0 | 4 | 0 | 1 | 4 | 3 | 4 | 4 | 4 |
| 3 | 1 | 0 | 4 | 0 | 1 | 4 | 2 | 4 | 4 | 4 |
|  | 2 | 0 | 4 | 0 | 1 | 4 | 2 | 4 | 4 | 4 |
| Score moyen |  | 0 | 4 | 0 | 1 | 4 | 2,66 | 4 | 4 | 4 |

**Résultats des essais d'auto et co-agrégation entre UPEC2 et les levures**

Capacité d'auto-agrégation des levures et des souches UPEC

Les isolats de levure vivante (GI), UPEC1 et UPEC2 ne sont pas capables de s'auto-agréger, le score obtenu pour chacun de ces isolats étant de 0.

Les isolats de *S. boulardii* (SCB) montrent une capacité limitée d'auto-agrégation, le score obtenu pour ces isolats étant de 1.

Les isolats de levure inactive (IY) et de glucanes pariétaux (WG) montrent une forte auto-agrégation, le score obtenu pour chacun de ces isolats étant de 4.

Capacité des levures à induire l'agrégation avec les souches UPEC

Les isolats de IY, GI, SCB et WG sont capables d'agréger les 2 isolats d'*E. coli,* UPEC1 et UPEC2.

En particulier, l'isolat UPEC2 a un score de co-agrégation maximal avec GI, SCB et WG suivi de IY avec un score légèrement plus faible (cf. Tableau 2). L'isolat de UPEC1 a un score maximal avec SCB, WG suivi de GI et IY avec un score légèrement plus faible (cf. Tableau 1).

*Conclusion*

La capacité d'induire la co-agrégation avec *E.coli* peut constituer un phénomène important dans l'établissement et le maintien d'une flore urogénitale saine et un mécanisme de défense important contre les infections urogénitales.

Le présent exemple démontre ainsi que :

- Tous les composés testés sont capables d'induire la co-agrégation avec les souches UPEC 1 et UPEC 2
- Les levures SCB et WG sont capables de co-agréger avec UPEC 1 et UPEC 2 avec un score maximal ;
- Les levures GI et IY sont capables de co-agréger avec UPEC 1 et UPEC 2.

Ainsi, des probiotiques peuvent co-agréger avec des souches multirésistantes aux antibiotiques. Il y a donc une réelle utilisation bénéfique des levures en remplacement partiel ou en complément d'une antibio-thérapie pour aider lors du traitement d'infections urinaires multirésistants aux antibiotiques.

**Exemple 2 : Evaluation de l'effet de levures sur la colonisation de l'intestin murin par une souche uropathogène**

*Matériel & méthodes*

Modèle murin

Le modèle animal utilisé est une souris femelle de lignée BALB/cYJ, âgée de 7 semaines. Les souris sont logées par groupes de 5 sur un support ventilé.

Souche uropathogène

La souche uropathogène utilisée dans l'exemple est une souche *E. coli* uropathogène multirésistante aux antibiotiques, appelée ci-après UPEC3.

Levures testées

Les levures testées sont les suivantes :

- Levure inactive *Saccharomyces cerevisiae* CNCMI-3856 (IY) ayant un taux de matière sèche $\geq$ 94% et un pH compris entre 5,5 et 6,3,
- Levure vivante *Saccharomyces cerevisiae* CNCM I-3856 (GI) à une teneur $\geq$ 5.10$^9$ UFC/g.

Protocole

J (= Jour) - 2, les fèces sont collectées et du sulfate de streptomycine (5mg/mL) est ajouté pendant 2 jours à l'eau de boisson pour diminuer les bactéries aérobies/anaérobies résidentes. La récolte de fèces est réalisée en plaçant chaque animal dans des boîtes vides le temps nécessaire à la collecte de 2 à 4 pellets fécaux, lesquels sont ensuite transférés dans des tubes stériles, pré-pesés, de 2 mL. Les tubes sont ensuite pesés et les échantillons fécaux sont resuspendus dans 1 mL de PBS, dilués en série au 1 :10, et déposés sur des boites de milieu gélosé de Drigalski additionnées de 100 $\mu$g/ml de Streptomycine et de Kanamycine.

J = 0, inoculation de la souche UPEC3

Avant l'inoculation bactérienne, les fèces des souris sont collectées pour vérifier l'absence d'entérobactéries résistantes à la streptomycine et à la kanamycine.

L'inoculation de la souche UPEC3 est réalisée par gavage oral à une dose de $1 \times 10^7$ UFC. L'inoculât est issu d'une culture d'une nuit en milieu liquide LB (pour « Lysogeny Broth » ou bouillon lysogène), diluée ensuite dans 200$\mu$L

d'une solution de bicarbonate de sodium.

En même temps, l'eau de boisson a été remplacée par une solution aqueuse de 1 mg/mL de sulfate de kanamycine pendant 2 jours.

J = 1, début du traitement expérimental administré quotidiennement par gavage, comme il suit :

- Groupe 1 (contrôle) : 10 souris divisées en 2 cages reçoivent 200 $\mu$L de PBS ;
- Groupe 2 (IY) : 10 souris divisées en 2 cages reçoivent 1 mg du composé IY re-suspendu dans 200 $\mu$L de PBS ;
- Groupe 3 (GI) : 10 souris divisées en 2 cages reçoivent 1 mg du composé GI re-suspendu dans 200 $\mu$L de PBS ;

J = 14, aucun traitement n'est administré.

Les animaux sont sacrifiés et disséqués pour prélever les sections intestinales iléales et du colon. Lors du prélèvement, les organes sont vidés de leur contenu et lavés dans du PBS stérile dans des conditions aseptiques.

Les sections intestinales pesées sont homogénéisées dans du PBS au gentle MACS™ Octo Dissociator et diluées de 10 en 10 dans des plaques de microtitrations. Un aliquote de chaque dilution est ensuite déposé sur une boite de milieu gélosé de Drigalsky additionnées de 100 $\mu$g/mL de streptomycine et kanamycine pour l'énumération des souches UPEC3 adhérentes. Ce dépôt est réalisé en triple. Le comptage est effectué manuellement.

*Résultats*

Une souris du groupe 3 (GI) est morte au jour 7 sans cause précise mais probablement dû au stress du gavage répété.

Une section intestinale du colon d'une souris du groupe 2 (IY) n'a pas été utilisable.

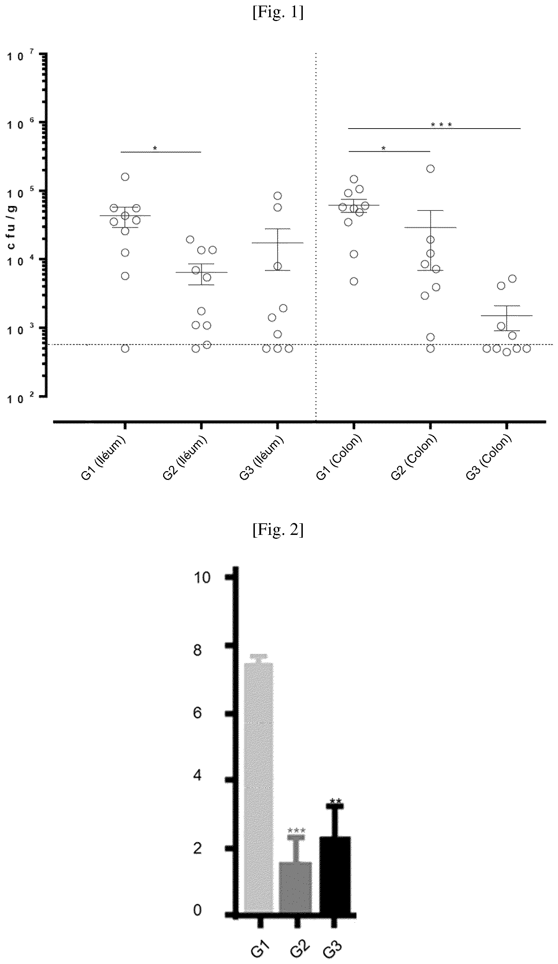

La figure 1 représente la quantité en UFC de la souche UPEC3 dans les sections intestinales pour chacun des groupes testés au jour 14. (* $p < 0.05$; *** $p < 0,0001$)

On observe une diminution significative (test Mann-Whitney) de la quantité de la souche UPEC3 dans la section iléale de l'intestin pour le groupe 2 (IY).

On observe une réduction significative de la quantité de la souche UPEC3 dans le colon pour les groupes 2 (IY) et 3 (GI).

*Conclusion*

Le niveau de la flore adhérente dans l'iléum et le colon est significativement plus élevé dans le groupe contrôle que dans les deux autres groupes ayant reçu les levures. Plus précisément, il peut être observé dans la section du colon que les deux produits conduisent à une réduction significative du taux de souches UPEC3 adhérentes, alors que dans les sections iléales seul le produit IY a un effet significatif. Cependant, avec le produit GI, l'effet dans la section iléale est presque significatif car pour 4 souris sur 9 le taux des souches UPEC3 adhérentes a atteint le seuil de détection.

Les deux produits, levure vivante et inactive, présentent donc la capacité de réduire le niveau de la souche uropathogène d'*E. coli* adhérant à l'épithélium intestinal.

Ainsi ces deux levures ont la capacité à compromettre le maintien du réservoir intestinal de la souche UPEC3.

**Exemple 3 : Evaluation de l'efficacité anti-infectieuse d'une levure**

*Matériel et méthodes*

Souche E. coli

Une souche *Escherichia coli* uropathogène, dite UPEC4 dans le présent exemple, est utilisée.

Composés testés

Tampon

La solution tampon utilisée est une solution tampon PBS 1X

Levure

La levure évaluée est la levure vivante *Saccharomyces cerevisiae* CNCM I-3856 sous forme sèche, appelée GI ci-après. Avant réhydratation la levure se présente sous une forme sèche et comprend une teneur $\geq 5.10^9$ UFC/g.

Avant les tests, la levure est réhydratée avec le tampon PBS.

Tobramycine

La tobramycine est utilisée car c'est un antibiotique puissant et un médicament de référence. Avant les tests, la tobramycine est suspendue avec le tampon PBS.

Modèle animal

Le modèle animal utilisé est une souris femelle de lignée C57BL/6J, âgée de 8 semaines. Les souris sont logées par groupes de 5 sur une litière à copeaux à une température d'environ 22°C. Elles sont nourries avec un aliment complet d'entretien standard tel que SAFE A04® pour rats et souris et ont accès à l'eau *ad libitum.*

Groupes tests

10 souris femelles par groupe sont utilisées (sauf le groupe 1, seulement 5 femelles), âgées de 8 semaines.
Les groupes testés sont les suivants :

G1 :

$$\text{souris + UPEC4 + tampon = contrôle ;}$$

G2:

$$\text{souris + UPEC4 + Tobramycine (30mg/kg) = contrôle positif ;}$$

G3 :

$$\text{souris + UPEC4 + GI (13,5mg/kg).}$$

Protocoles

Administration des composés à tester

Les composés sont administrés par voie locale (administration transurétrale), 24 heures et 1 heure avant l'infection pour les groupes 1 et 3. La tobramycine, médicament de référence, est administré par voie intrapéritonéale 1 heure avant l'infection dans le groupe 3.

Infection des souris avec UPEC4

La souris est tout d'abord anesthésiée sous Isoflurane (3%). L'urine est évacuée de la vessie avant l'infection. Puis, la souris est infectée avec $50\mu L$ de UPEC4 (administrés en 2-3 secondes) soit une administration de $6\times10^7$ UFC (Unité Formant Colonie) de UPEC4 par voie transurétrale.
L'infection par voie transurétrale est réalisée en insérant un tube stérilisé en polyéthylène (dimension interne de 0,28mm, dimension externe de 0,61mm) attaché à une aiguille hypodermique de 30g dans l'urètre.
20 heures après l'infection, des échantillons d'urine sont collectés sur chaque souris. La vessie et les reins sont retirés et homogénéisés pour le dénombrement des UFC.

Préparation des échantillons en vue de la détermination de la charge bactérienne (UFC)

Les animaux sont sacrifiés 20 heures après l'inoculation de la souche UPEC par inhalation de $CO_2$. Le rein droit et la moitié de la vessie sont homogénéisés dans une solution saline stérile isotonique utilisant le système Dispomix® d'homogénéisation à usage unique stérile. Les dilutions en cascade au 1/10 des échantillons (urine, homogénat de rein et vessie) sont réalisées avec une solution saline isotonique. Un aliquote de 10 $\mu l$ de chaque dilution est ensuite déposé sur une boite de milieu gélosé d'agar BHI (pour « Brain Heart Infusion »). Ce dépôt est réalisé en double. La boite de pétri est ensuite incubée une nuit (overnight) à 37°C afin de déterminer le nombre de bactéries viables en UFC. Le comptage est effectué manuellement par deux expérimentateurs.

Analyse statistique des résultats

Avant le lancement de l'analyse statistique, le test d'Agostino et Pearson a été réalisé afin de déterminer si les variables suivent la loi normale.

L'évaluation statistique des différences entre les groupes expérimentaux est déterminée en utilisant des tests paramétriques (Anova à un facteur suivi par un test post-hoc de Dunnett, pour des données distribuées normalement) ou non paramétriques (Kruskal Wallis suivi par une comparaison multiple du test de Dunn pour une distribution anormale) selon les résultats du test de Normalité.

Le test de Grubb est utilisé pour déterminer si une des valeurs dans le groupe est une valeur aberrante significative.

Les valeurs sont exprimées comme la moyenne $\pm$ SEM. Une valeur de $P \leq 0,05$ est considérée comme statistiquement significative.

*Résultats*

La figure 2 représente la charge bactérienne en UFC dans l'urine pour chacun des groupes testés (n.s : non significatif, *p<0,05, *** p<0,001 pour G2 et G3 comparés à G1).

On observe une diminution significative des UFC dans l'urine chez les souris infectées avec UPEC4 et traitées avec GI (groupe 3) et la Tobramycine (groupe 2) par rapport aux souris du groupe 1 infectées avec UPEC4.

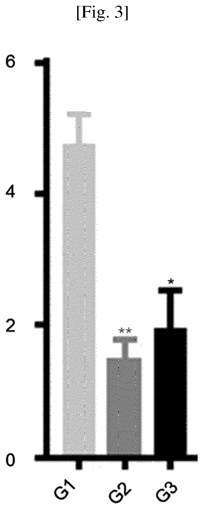

La figure 3 représente la charge bactérienne en UFC dans la vessie pour chacun des groupes testés (n.s : non significatif, *p<0,05, *** p<0,001 pour G2 et G3 comparés à G1).

On observe une diminution significative des UFC dans la vessie chez les souris infectées avec UPEC4 et traitées avec GI (groupe 3) et la tobramycine (groupe 2) par rapport aux souris du groupe 1 infectées avec UPEC4.

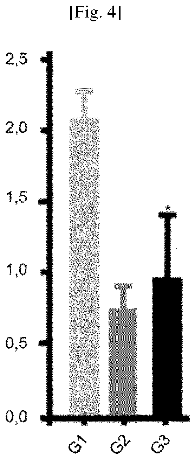

La figure 4 représente la charge bactérienne en UFC dans les reins pour chacun des groupes testés (n.s : non significatif, *p<0,05, *** p<0,001 pour G2 et G3 comparés à G1).

On observe une diminution significative des UFC dans les reins chez les souris infectées avec UPEC4 et traitées avec GI (groupe 3) et la tobramycine (groupe 2) par rapport aux souris du groupe 1 infectées avec UPEC4.

*Conclusion*

La souche UPEC4 dans le modèle de souris induit une augmentation significative de la charge bactérienne.

L'administration de la levure vivante *Saccharomyce cerevisiae* CNCM I-3856 par voie locale chez des souris infectées par une souche UPEC4 induit une diminution significative de la charge bactérienne dans l'urine, la vessie, les reins.

L'administration locale de la levure vivante *Saccharomyce cerevisiae* CNCM I-3856 inhibe donc de manière très active l'infection du tractus urinaire par la souche *Escherichia coli* uropathogène.

**Revendications**

1. Composition comprenant au moins une levure pour son utilisation dans la prévention de la cystite simple et/ou récidivante **caractérisée en ce que** la levure est sous la forme d'une levure vivante, une levure morte, d'écorces de levure, de fractions purifiées d'écorces de levure ou leurs mélanges, et **en ce que** la levure est la levure obtenue par culture de la souche *Saccharomyces cerevisiae* déposée à la CNCM le 17 octobre 2007 sous le numéro I-3856.

2. Composition comprenant au moins une levure pour son utilisation dans la prévention de la cystite simple et/ou récidivante **caractérisée en ce que** la levure est sous la forme d'une levure vivante, une levure morte, d'écorces de levure, de fractions purifiées d'écorces de levure ou leurs mélanges, et **en ce que** la levure est la levure obtenue par culture de la souche *Saccharomyces cerevisiae* var. *boulardii* déposée à la CNCM le 21 août 2007 sous le numéro I-3799.

3. Composition comprenant au moins une levure pour son utilisation dans la prévention de la cystite simple et/ou récidivante selon la revendication 1 ou la revendication 2, **caractérisée en ce que** la cystite simple et/ou récidivante est une cystite induite par des bactéries *Escherichia coli* uropathogènes.

**4.** Composition comprenant au moins une levure pour son utilisation dans la prévention de la cystite simple et/ou récidivante selon l'une quelconque des revendications 1 à 3, **caractérisée en ce que** la levure est sous forme de levure sèche.

**5.** Composition comprenant au moins une levure pour son utilisation dans la prévention de la cystite simple et/ou récidivante selon l'une quelconque des revendications 1 à 4, **caractérisée en ce que** la composition est destinée à une administration locale ou orale.

**6.** Composition comprenant au moins une levure pour son utilisation dans la prévention de la cystite simple et/ou récidivante selon l'une quelconque des revendications 1 à 5, **caractérisée en ce que** la composition est sous forme de gélule, pilule, pastille, comprimé, capsule, poudre, suspension, solution liquide, granule, ovule, gel ou crème.

**7.** Composition comprenant au moins une levure pour son utilisation dans la prévention de la cystite simple et/ou récidivante selon l'une quelconque des revendications 1 à 4, **caractérisée en ce que** la composition est une composition alimentaire ou un complément alimentaire.

**8.** Composition comprenant au moins une levure pour son utilisation dans la prévention de la cystite simple et/ou récidivante selon l'une quelconque des revendications 1 à 7, **caractérisée en ce que** la composition comprend un autre ingrédient actif choisi parmi un extrait de canneberge, myrtilles, arbousier, airelle rouge, bruyère ou leurs mélanges lorsqu'elle est destinée à une administration orale.

**9.** Dispositif médical **caractérisé en ce qu'**il comprend une composition selon l'une quelconque des revendications 1 à 4, pour son utilisation dans la prévention de la cystite simple et/ou récidivante.

## Patentansprüche

**1.** Zusammensetzung, die wenigstens eine Hefe umfasst, zur Verwendung bei der Prävention von einfacher und/oder rezidivierender Zystitis, **dadurch gekennzeichnet, dass** die Hefe in Form einer lebenden Hefe, einer toten Hefe, von Heferinden, gereinigten Fraktionen von Heferinden oder deren Mischungen vorliegt, und dass die Hefe jene Hefe ist, die durch Kultivierung des Stammes Saccharomyces cerevisiae erhalten wird, der am 17. Oktober 2007 unter der Nummer I-3856 bei der CNCM hinterlegt wurde.

**2.** Zusammensetzung, die wenigstens eine Hefe umfasst, zur Verwendung bei der Prävention von einfacher und/oder rezidivierender Zystitis, **dadurch gekennzeichnet, dass** die Hefe in Form einer lebenden Hefe, einer toten Hefe, von Heferinden, gereinigten Fraktionen von Heferinden oder deren Mischungen vorliegt, und dass die Hefe jene Hefe ist, die durch Kultivierung des Stammes Saccharomyces cerevisiae erhalten wird, der am 21. August 2007 unter der Nummer I-3799 bei der CNCM hinterlegt wurde.

**3.** Zusammensetzung, die wenigstens eine Hefe umfasst, zur Verwendung bei der Prävention von einfacher und/oder rezidivierender Zystitis nach Anspruch 1 oder 2, **dadurch gekennzeichnet, dass** die einfache und/oder rezidivierende Zystitis eine durch uropathogene Escherichia coli-Bakterien induzierte Zystitis ist.

**4.** Zusammensetzung, die wenigstens eine Hefe umfasst, zur Verwendung bei der Prävention von einfacher und/oder rezidivierender Zystitis nach einem der Ansprüche 1 bis 3, **dadurch gekennzeichnet, dass** die Hefe in Form von Trockenhefe vorliegt.

**5.** Zusammensetzung, die wenigstens eine Hefe umfasst, zur Verwendung bei der Prävention von einfacher und/oder rezidivierender Zystitis nach einem der Ansprüche 1 bis 4, **dadurch gekennzeichnet, dass** die Zusammensetzung für die lokale oder orale Verabreichung bestimmt ist.

**6.** Zusammensetzung, die wenigstens eine Hefe umfasst, zur Verwendung bei der Prävention von einfacher und/oder rezidivierender Zystitis nach einem der Ansprüche 1 bis 5, **dadurch gekennzeichnet, dass** die Zusammensetzung in Form einer Gelatinekapsel, Pille, Pastille, Tablette, Kapsel, eines Pulvers, einer Suspension, einer flüssigen Lösung, eines Granulats, eines Ovulums, eines Gels oder einer Creme vorliegt.

**7.** Zusammensetzung, die wenigstens eine Hefe umfasst, zur Verwendung bei der Prävention von einfacher und/oder rezidivierender Zystitis nach einem der Ansprüche 1 bis 4, **dadurch gekennzeichnet, dass** die Zusammensetzung eine Lebensmittelzusammensetzung oder ein Nahrungsergänzungsmittel ist.

**8.** Zusammensetzung, die wenigstens eine Hefe umfasst, zur Verwendung bei der Prävention von einfacher und/oder rezidivierender Zystitis nach einem der Ansprüche 1 bis 7, **dadurch gekennzeichnet, dass** die Zusammensetzung einen weiteren Wirkstoff umfasst, der aus einem Extrakt aus Cranberry, Heidelbeeren, Erdbeerbaum, Preiselbeeren, Heidekraut oder deren Mischungen gewählt ist, wenn sie für die orale Verabreichung bestimmt ist.

**9.** Medizinische Vorrichtung, **dadurch gekennzeichnet, dass** sie eine Zusammensetzung nach einem der Ansprüche 1 bis 4 zur Verwendung bei der Prävention von einfacher und/oder rezidivierender Zystitis umfasst.

**Claims**

**1.** A composition comprising at least one yeast for use in the prevention of simple and/or recurrent cystitis, **characterized in that** the yeast is in the form of a live yeast, a dead yeast, yeast cell walls, purified fractions of yeast cell walls or mixtures thereof, and **in that** the yeast is the yeast obtained by culture of the *Saccharomyces cerevisiae* strain deposited with the CNCM on 17 October 2007 under number 1-3856.

**2.** A composition comprising at least one yeast for use in the prevention of simple and/or recurrent cystitis, **characterized in that** the yeast is in the form of a live yeast, a dead yeast, yeast cell walls, purified fractions of yeast cell walls or mixtures thereof, and **in that** the yeast is the yeast obtained by culture of the *Saccharomyces cerevisiae var. boulardii* deposited with the CNCM on 21 August 2007 under number 1-3799.

**3.** The composition comprising at least one yeast for use in the prevention of simple and/or recurrent cystitis as claimed in claim 1 or claim 2, **characterized in that** the simple and/or recurrent cystitis is a cystitis induced by uropathogenic *Escherichia coli* bacteria.

**4.** The composition comprising at least one yeast for use in the prevention of simple and/or recurrent cystitis according to any one of the claims 1 to 3, **characterized in that** the yeast is in the form of dry yeast.

**5.** The composition comprising at least one yeast for use in the prevention of simple and/or recurrent cystitis as claimed in any one of claims 1 to 4, **characterized in that** the composition is intended for local or oral administration.

**6.** The composition comprising at least one yeast for use in the prevention of simple and/or recurrent cystitis as claimed in any one of claims 1 to 5, **characterized in that** the composition is in the form of a hard capsule, pill, pastille, tablet, soft capsule, powder, suspension, liquid solution, granules, pessary, gel or cream.

**7.** The composition comprising at least one yeast for use in the prevention of simple and/or recurrent cystitis as claimed in any one of claims 1 to 4, **characterized in that** the composition is a food composition or a dietary supplement.

**8.** The composition comprising at least one yeast for use in the prevention of simple and/or recurrent cystitis as claimed in any one of claims 1 to 7, **characterized in that** the composition comprises another active ingredient selected from an extract of cranberry, bilberry, arbutus, red cranberry, heather or mixtures thereof when it is intended for oral administration.

**9.** A medical device, **characterized in that** it comprises a composition as claimed in any one of claims 1 to 4, for use thereof in the prevention of simple and/or recurrent cystitis.

[Fig. 1]

[Fig. 2]

[Fig. 3]

[Fig. 4]

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2009103884 A

- WO 2014009656 A

**Littérature non-brevet citée dans la description**

- **RAMIRO J. MADDEN-FUENTES et al.** *Clinical Therapeutics,* 2015, vol. 37 (9
- **ALKIL.** *Pediatr. Nephrol,* 2006

- **G. REED ; T.W. NAGODAWITHANA.** Yeast Technology. Van Nostrand Reinhold, 1991
- **G. REED ; T.W. NOGODAWITHANA.** Yeast Technology. Van Nostrand Reinhold, 1991